## Europäisches Patentamt
## European Patent Office
## Office européen des brevets

(19)

(11) Veröffentlichungsnummer: **0 139 855**
**A1**

(12) # EUROPÄISCHE PATENTANMELDUNG

(21) Anmeldenummer: **84107862.9**

(22) Anmeldetag: **05.07.84**

(51) Int. Cl.⁴: **A 61 M 31/00**

(30) Priorität: **08.07.83 DE 3324780**

(43) Veröffentlichungstag der Anmeldung:
**08.05.85 Patentblatt 85/19**

(84) Benannte Vertragsstaaten:
**AT BE CH DE FR GB IT LI LU NL SE**

(71) Anmelder: **PLANTORGAN WERK Heinrich G.E. Christensen KG
Hornbusch 1
D-2903 Bad Zwischenahn(DE)**

(72) Erfinder: **Fink, Ernst, Prof. Dr. med. Dr. rer. nat.
Stellhorner Strasse 1
D-2910 Westerstede-Giesselhorst(DE)**

(74) Vertreter: **Kinzebach, Werner, Dr. et al,
Patentanwälte Reitstötter, Kinzebach und Partner
Postfach 780
D-8000 München 43(DE)**

(54) **Vorrichtung zur intra-analen Applikation von flüssigen oder pastösen Wirkstoffzubereitungen.**

(57) Vorrichtung zur intra-analen Applikation von flüssigen oder pastösen Wirkstoffzubereitungen, mit einem im wesentlichen schlank-kegelförmigen Applikator (10) mit Austrittsöffnungen (50), wobei der Applikator (10) mit einem Behälter (20) für die zu applizierende Wirkstoffzubereitung verbunden ist und Betätigungsmittel (40) aufweist, bei deren Betätigung die Wirkstoffzubereitung aus den Austrittsöffnungen (50) gepreßt wird.

FIG.4

EP 0 139 855 A1

Croydon Printing Company Ltd.

Die Erfindung betrifft eine Vorrichtung zur intra-analen Applikation von flüssigen oder pastösen Wirkstoffzubereitungen, die mit einem im wesentlichen schlank-kegelförmigen Applikator mit Austrittsöffnungen ausgestattet ist.

Bei Erkrankungen oder Verletzungen im Analbereich ist es notwendig, Wirkstoffzubereitungen zu applizieren, wobei gerade dieser Bereich vom Patienten selbst relativ schwer zu behandeln ist. Insbesondere ist es notwendig, bei Applikation, zum Beispiel von Hämorrhoidal-Salbe im Sphinkter-Bereich, eine starke Dehnung der Sphinktermuskulatur zu erzielen, damit die Salbe die sonst unzugänglichen Krypten und Fissuren erreichen kann. Um dies· zu erreichen, wurde eine schlank-kegelförmige Kanüle mit abgerundeter Spitze entwickelt, die an ihrem Ende mit einem Gewinde versehen ist. Diese Kanüle wird auf eine im Handel erhältliche Tube mit Salbenfüllung aufgesetzt, wenn man den Wirkstoff applizieren will.

Bei der bekannten Vorrichtung treten nun mehrere Nachteile auf. Zum einen verbleibt nach der Applikation der Salbe im Applikator ein Rest des Wirkstoffes, so daß dieser, insbesondere dann, wenn er relativ dünnflüssig ist, nach Abnehmen des Applikators von der Tube und nach dem Verschließen der Tube mit der dazugehörigen Verschlußkappe, ausläuft. Zum anderen wird der Applikator über einen längeren Zeitraum hinweg benutzt, so daß im Laufe der Zeit ein erhebliches Keimwachstum auf dem Applikator stattfindet, wodurch die erkrankten Stellen immer wieder infiziert werden. Darüber hinaus ist es für den Patienten, der die Wirkstoffzubereitung selbst appliziert, nahezu unmöglich, eine genaue, der wirksamen Dosis entsprechende Menge zu applizieren, da die optische Kontrolle fehlt.

Ausgehend vom oben genannten Stand der Technik ist es Aufgabe der vorliegenden Erfindung, eine Vorrichtung aufzuzeigen, mit der eine genaue Dosierung der Wirkstoffzubereitung bei gleichzeitigem Verhindern des Keimwachstumes auf dem Applikator gegeben ist.

Diese Aufgabe wird durch einen Applikator nach dem Hauptanspruch gelöst, bevorzugte Ausführungsformen ergeben sich aus den Unteransprüchen und den nachfolgenden Ausführungsbeispielen.

Der erfindungsgemäße Applikator kann kostengünstig aus Kunststoff gefertigt werden und in einer der geplanten Behandlungsdauer entsprechenden Anzahl in Packungen in den Handel gelangen. Auf diese Weise ist es für den Patienten möglich, die Dosierungsvorschriften des behandelnden Arztes einzuhalten und dabei gleichzeitig, ohne Maßnahmen zur Sterilisierung des Applikators treffen zu müssen, im wesentlichen vollständig keimfrei zu arbeiten.

Im folgenden wird die vorliegende Erfindung anhand von Ausführungsbeispielen näher beschrieben, zu deren Verständnis die beiliegenden Abbildungen dienen sollen. Hierbei zeigt

Figur 1    eine bevorzugte Ausführungsform des Applikators mit angegossener Kunststofftube im Teil-Längsschnitt,

Figur 2    eine Teil-Seitenansicht des Applikators nach Figur 1,

Figur 3    eine weitere bevorzugte Ausführungsform des Applikators mit angegossener Kunststofftube im Längsschnitt,

Figur 4     eine weitere Ausführungsform des Applikators
            in Kolben-Zylinderausführung,

Figur 5     eine weitere bevorzugte Ausführungsform des
            Applikators in Kolben-Zylinderausführung,

Figur 6     einen Längsschnitt durch einen Applikator
            mit einem darin enthaltenden Zylinder,

Figur 7     einen Längsschnitt durch eine Betätigungsvorrichtung für den Applikator nach Figur 6,

Figur 8     einen Horizontalschnitt entlang der Linie
            VIII-VIII aus den Figuren 3 und 5,

Figur 9     einen Horizontalschnitt entlang der Linie
            IX-IX aus den Figuren 3 und 5,

Figur 10    eine Seitenansicht des Endabschnittes einer
            weiteren Ausführungsform des Applikators mit
            Zylinder-Kolbenvorrichtung, und

Figur 11    einen Längsschnitt durch eine weitere bevorzugte Ausführungsform des Applikators.

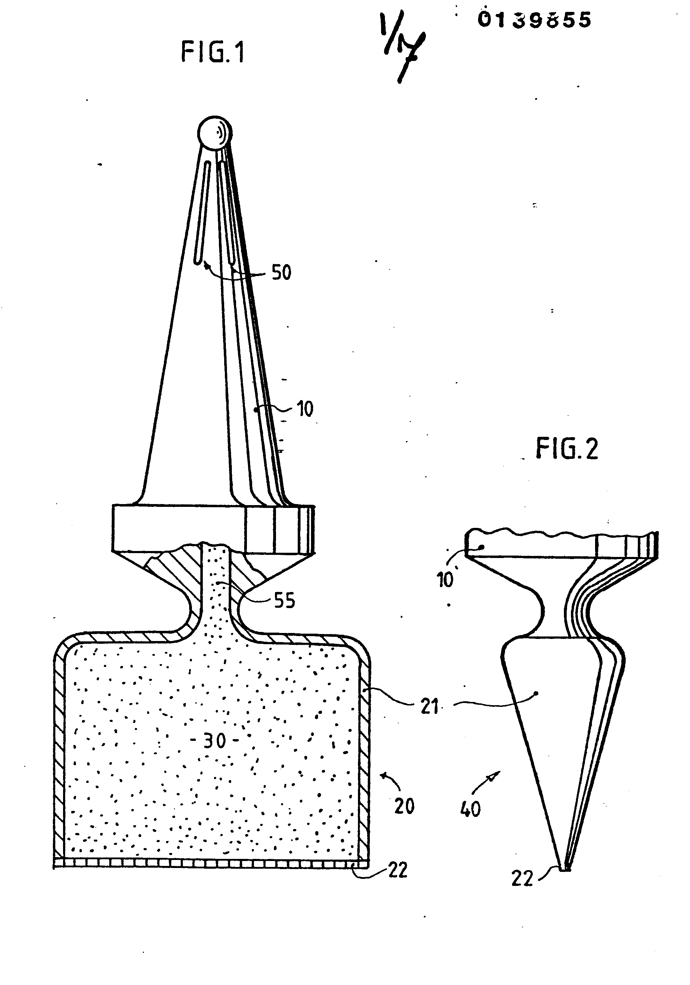

Der Applikator nach den Figuren 1 und 2 weist einen kegelförmigen Applikatorteil 10 auf, dessen Spitze kugelförmig abgerundet ist. Auf der Mantelfläche, in der Nähe
der Spitze, befinden sich schlitzförmige Austrittsöffnungen 50 für die Salbe, die über einen Kanal 55 mit
dem Innenraum einer an den Applikator angegossenen Tube
21 in Verbindung stehen. An seinem, der abgerundeten
Spitze abgewandten Ende weiste der Applikator 10 eine
Verdickung auf, welche die maximale Eindringtiefe des Applikators begrenzt. Zwischen dieser Verdickung und der Tube verengt sich der Applikator in einem Hals, so daß die Tube 21, die den Behälter 20 für die Wirkstoffzubereitung 30 darstellt, ein umgrenztes Kissen darstellt. Hierbei werden die Betätigungsmittel 40 durch die flachen Seiten der Tube gebildet, die an ihrem unteren Rand nach dem Befüllen an einem Abschlußrand 22 unter Druck thermisch verschweißt wird. Vorzugsweise werden vor dem Befüllen die Austrittsöffnungen 50 in herkömmlicher Weise, zum Beispiel durch Release-Haftfolien verschlossen, die vor dem Gebrauch abgezogen werden können. Dadurch, daß die Tube 21 durch den Hals im Übergang zum Applikator 10 genau umgrenzte Außenumrisse aufweist, kann der Benutzer den Applikator zweimal verwenden, und dabei jedes Mal die genau richtige Dosierung erzielen, indem er bei der ersten Anwendung nur die eine Längshälfte der Tube zusammendrückt, bei der zweiten Anwendung die Tube jedoch ganz ausquetscht. Selbstverständlich muß der Tubeninhalt demzufolge die doppelte Menge einer einfachen Dosis enthalten. Darüber hinaus kann man davon ausgehen, daß die zwei Applikationen so kurz hintereinander erfolgen, daß ein merkliches Keimwachstum noch nicht stattgefunden hat.

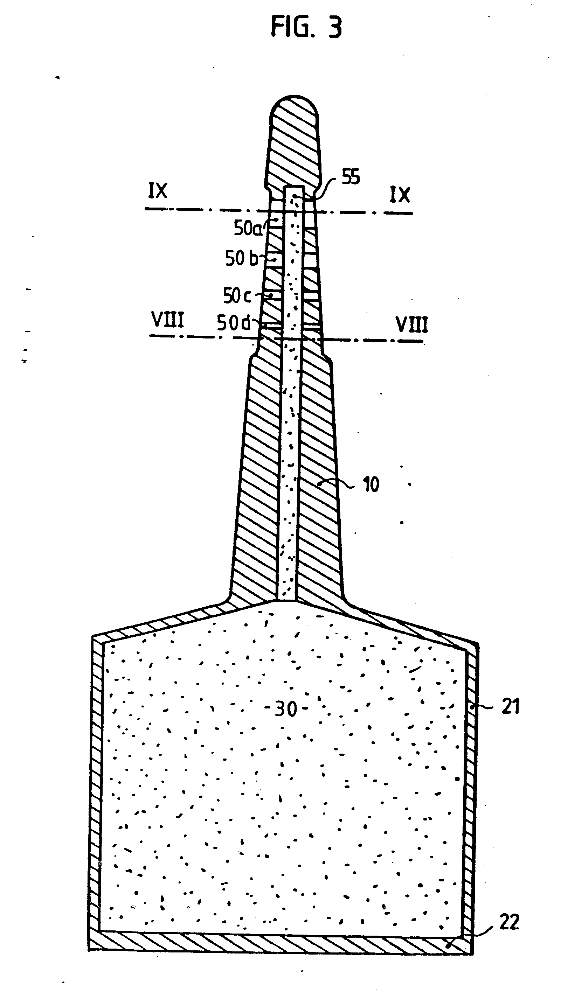

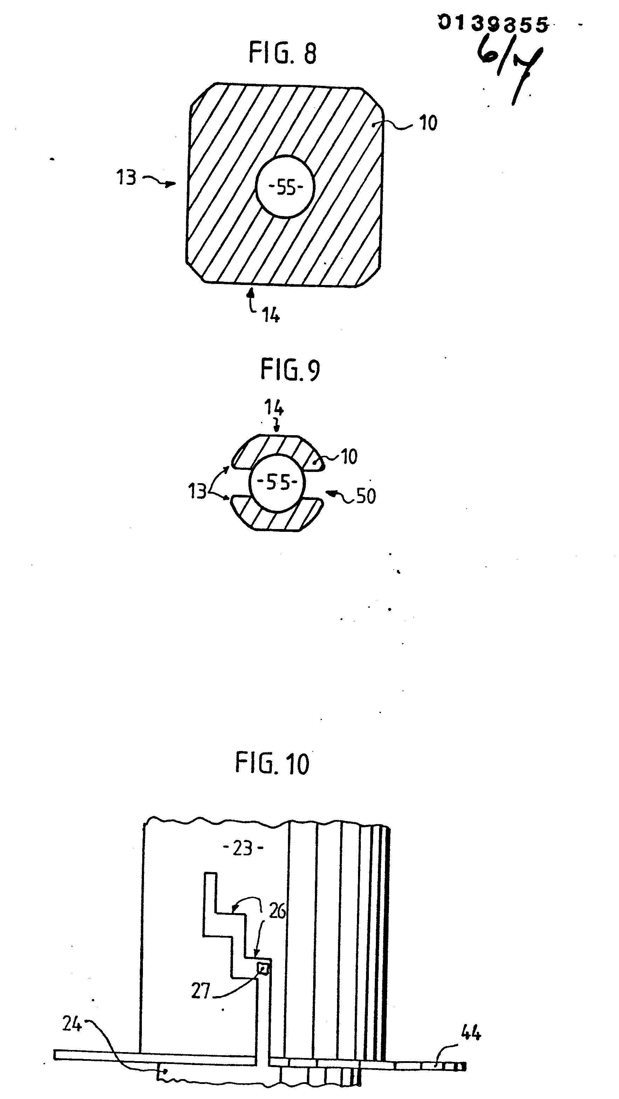

Bei der weiteren bevorzugten Ausführungsform der erfindungsgemäßen Vorrichtung nach Figur 3 bildet der obere Tubenrand selbst die Begrenzung zum Applikator 10; gegenüber der Ausführungsform nach den Figuren 1 und 2 wird bei dieser Ausführungsform eine Materialersparnis erzielt. Weiterhin ist aus Figur 3 zu erkennen, daß die Austrittsöffnungen, die über den Kanal 55 mit dem Innenraum der Tube 21 in Verbindung stehen, in Vertiefungen der Mantelfläche des Applikators münden, wie dies auch aus den Horizontalschnitten nach den Figuren 8 und 9 ersichtlich ist. Auf diese Weise ist gewährleistet, daß der Austritt der Salbe über einen weiteren Bereich der Applikatorspitze
hin gleichmäßig erfolgen kann. Weiterhin ist aus Figur
3 ersichtlich, daß die Austrittsöffnungen 50a bis 50d
verschiedene Querschnittsflächen aufweisen, und zwar
so, daß die der Tube 21 abgewandte Öffnung 50a den größten,
die der Tube 21 zugewandte Öffnung 50d den geringsten
Querschnitt aufweist. Durch diese zunehmende Querschnittsanordnung ist gewährleistet, daß bei Wirkstoffzubereitungen mit hoher Viskosität beim Zusammendrücken
der Tube 21 gleichmäßige Mengen an Wirkstoffzubereitung
aus allen Austrittsöffnungen hervorquellen, da die verschiedenen Strömungswiderstände in den Austrittsöffnungen
die Strömungswiderstände längs des Verbindungskanales
55 kompensieren. Man kann hierbei sowohl die Dimensionie
rungen der Austrittsöffnungen 50a bis 50d (bzw. die
Unterschiede in deren Dimensionierung) oder den Durchmesser des Verbindungskanals 55 entsprechend der Viskosität des Produktes 30 einstellen. Selbstverständlich
ist der gleiche Effekt auch bei der Ausführungsform der
Austrittsöffnungen 50 nach Figur 1 erzielbar, indem man
diese kontinuierlich schlitzförmigen Austrittsöffnungen
in Richtung auf die Spitze des Applikators hin breiter
werden läßt.

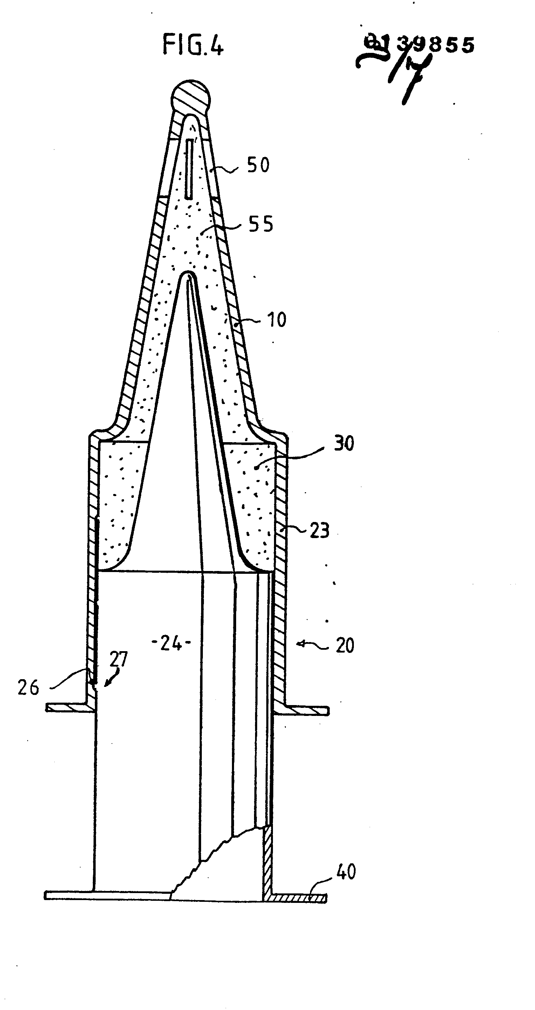

Eine weitere bevorzugte Ausführungsform der erfindungsgemäßen Vorrichtung ist in Figur 4 gezeigt. Hierbei ist
der Behälter 20 als Zylinder 23 ausgebildet, in dem ein
Kolben 24 sitzt. Der Zylinder 23 geht an seiner Spitze
in den Applikator mit Austrittsöffnungen 50 über. An
seinem, dem Applikator abgewandten Ende weist der Zylinder
23 einen flanschförmigen Rand auf, während der Kolben
24 an seinem Ende die Betätigungsmittel 40 (eine Abschlußplatte oder ein Flansch) trägt, so daß man den Zylinder
wie eine Injektionsspritze zwischen zwei Finger nimmt

:0139855

und mit dem Daumen auf die Betätigungsmittel 40 drücken kann. Aus Figur 4 geht weiterhin hervor, daß der Verbindungskanal 55 zu den Austrittsöffnungen 50 kegelförmig ausgebildet ist, wobei dieser Kegelform eine kegelförmige Spitze des Kolbens 24 entspricht. Durch diese absolut kongruente Formung von Zylinder/Applikator und Kolben ist gewährleistet, daß die gesamte Menge an Wirkstoffzubereitung 30, die in der Vorrichtung enthalten ist, bei Applikation abgegeben wird.

An dem in Figur 4 linken Rand des Zylinders 23 ist eine Nut mit Kerben 26 angebracht, auf dem Kolben 24 sind hierzu korrespondierende Nocken 27 vorgesehen. Diese Kerben-Nockenkombination bildet beim Hineindrücken des Kolbens 24 in den Zylinder 23 fühlbare Anschläge, so daß man diese Vorrichtung zur mehrmaligen Applikation von Wirkstoffen verwenden kann. Selbstverständlich werden die Abstände der Kerben zueinander so gewählt, daß der vom Kolben 24 im Zylinder 23 zurückgelegte Hub die einer wirksamen Dosis entsprechende Menge verdrängt.

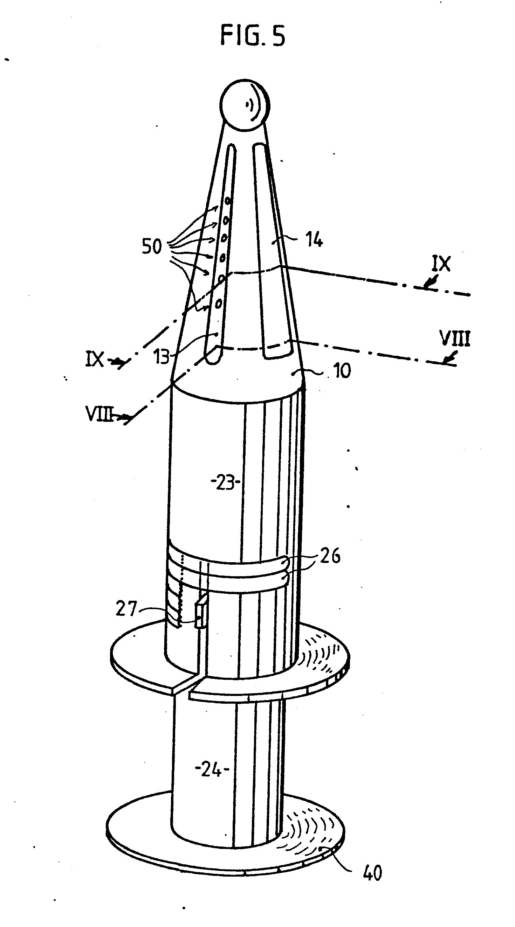

Eine weitere bevorzugte Ausführungsform der erfindungsgemäßen Vorrichtung mit Kolben-Zylinderkombination ist in Figur 5 gezeigt. Aus dieser Abbildung ist eine weitere bevorzugte Ausführungsform der Anschläge 26/27 ersichtlich: Der Zylinder 23 weist an seinem dem Applikator 10 abgewandten Ende einen Längs-Schlitz auf, in dem ein Nocken 27 geführt ist, der mit dem Kolben 24 einstückig verbunden ist. Der Schlitz im Zylinder 23 ist durch mehrere Abreißstreifen 26, die vorzugsweise aufgeklebt sind, teilweise abgedeckt, so daß der Kolben 24 immer nur so weit in den Zylinder 23 eingeschoben werden kann, bis der Nocken 27 an den letzten Abreißstreifen 26 stößt.

Bei der in Figur 5 gezeigten Vorrichtung sind drei Abreißstreifen bereits abgerissen, zwei weitere Abreißstreifen 26 können noch abgerissen werden, so daß noch eine zweimalige Applikation möglich ist. Es versteht sich hierbei von selbst, daß der durch einen Abreißstreifen 26 begrenzte Hub einer genau definierten Dosis an Wirkstoffzubereitung entspricht. Es ist darüber hinaus möglich, hinter dem Nocken 27 einen Verschlußstreifen anzubringen, so daß der Kolben 24 im Zylinder 23 nach der Befüllung fixiert ist.

Aus Figur 5 geht weiterhin hervor, daß zusätzlich zur Vertiefung 13 mit den darin angeordneten Austrittsöffnungen 50 eine weitere Vertiefung 14 auf der Mantelfläche des kegelförmigen Applikators 10 angebracht ist, die ebenfalls der besseren Verteilung des Wirkstoffes dient. Diese zusätzliche Vertiefung 14 ist noch einmal in den Horizontalschnitten nach den Figuren 8 und 9 (entsprechend den Schnittlinien IX-IX und VIII-VIII) gezeigt, die Anordnung der Austrittsöffnungen 50 innerhalb der Vertiefungen 13 geht aus diesen Schnittzeichnungen ebenfalls hervor.

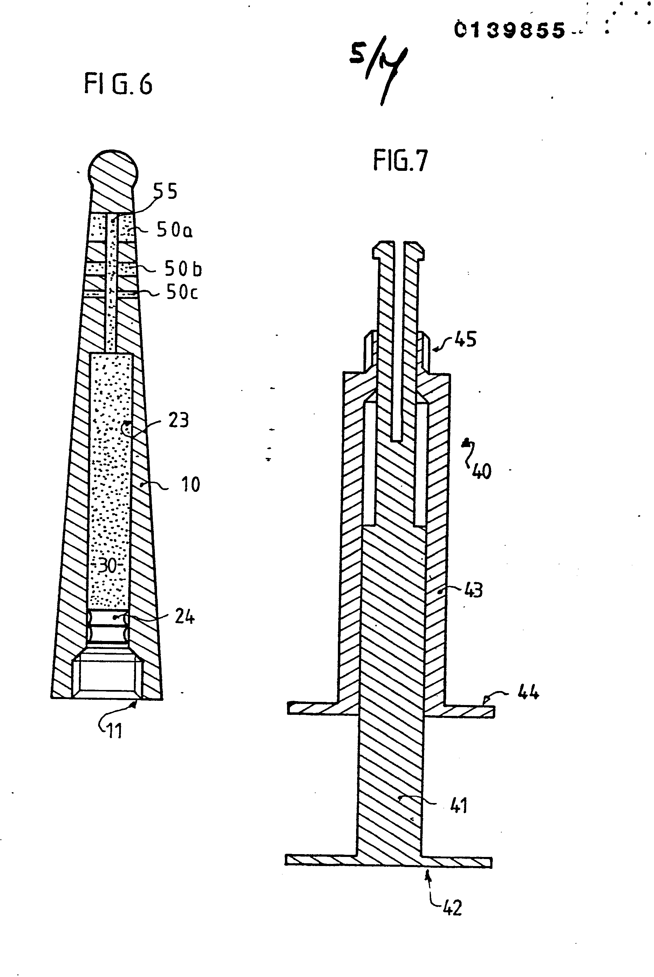

In den Figuren 6 und 7 ist eine weitere bevorzugte Ausführungsform der erfindungsgemäßen Vorrichtung gezeigt, wobei nach Figur 6 der Zylinder 23 im Applikator 10 angebracht und durch einen stopfenförmigen Kolben 24 verschlossen ist. Der Stopfen 24 wird hierbei vorzugsweise aus weich-elastischem Material gebildet, wie es auch bei Injektionsspritzen üblich ist. An seinem der Spitze abgewandten Ende weist der Applikator 10 ein Innengewinde 11 auf, mittels dessen der Applikator 10 auf die Betätigungsvorrichtung 40 bzw. auf deren Gewindeende 45 aufgeschraubt werden kann. Die Betätigungsvorrichtung 40 besteht aus einem Rohr 43 mit einer Fingerauflage 44 und aus einem Stempel 41 mit Daumenauflage 42. Der Stempel 41 ist an seinem Vorderende verjüngt und geschlitzt und weist an seiner Spitze nach außen ragende hakenförmige Fortsätze auf. Das verjüngte Ende des Stempels 41 ist so dimensioniert, daß es mit geringem Spiel im Vorderende des Rohres 43 gleitet, die hakenförmigen Fortsätze an der Spitze des Stempels sind so bemessen, daß man das verjüngte Ende des Stempels 41 in das Rohr 43 einsetzen kann, wobei durch den Schlitz die hakenförmigen Fortsätze zusammengepreßt werden und nach Durchqueren des Endes des Rohres 43 wieder auseinanderschnappen. Auf diese Weise ist gewährleistet, daß der Stempel 41 im Rohr 43 nur einen definierten Weg zurücklegen kann und nicht von selbst herausfällt. Weiterhin sind die hakenförmigen Fortsätze auf dem verjüngten Ende des Stempels 41 so bemessen, daß sie gerade in den Zylinder 23 passen. Wenn der Applikator 10 auf die Betätigungsvorrichtung 40 aufgeschraubt wird, so faßt man hierzu das Rohr 43 und den Applikator 10, so daß der Stempel 41 durch den Kontakt mit dem Kolben 24 nach hinten geschoben wird. Nach dem Aufschrauben des Innengewindes 11 des Applikators 10 auf das Außengewinde 45 des Rohres 43 sitzt somit das Ende des Stempels 41 (die Stirnfläche der hakenförmigen Fortsätze) auf dem Kolben 24 auf. Nach dem Applizieren (Niederdrücken des Stempels 41 relativ zum Rohr 43, wobei zwei Finger auf der Fingerauflage 44, der Daumen auf der Daumenauflage 42 liegen) schraubt man den leeren Applikator 10 ab und wirft ihn fort. Die Vorrichtung ist also zum einmaligen Gebrauch gedacht. Vorteilhafterweise kann man so eine Vielzahl von befüllten Applikatoren zusammen mit einer einzigen Betätigungsvorrichtung 40 verpacken, wodurch eine beträchtliche Ersparnis an Material erzielt werden kann und die Keimfreiheit im höchsten Maße gegeben ist. Hierbei ist es selbstverständlich möglich, die Betätigungsvorrichtung 40 auch in kinematisch umgekehrten Ausführungsformen vorzusehen. Darüber hinaus ist es auch möglich, statt der
mechanischen Betätigungsvorrichtung 40 eine pneumatische
Betätigungsvorrichtung ·in Form einer Aerosoldose vorzusehen, die den Kolben 24 mittels Treibgas in den Zylinder
23 drückt.

Figur 10 zeigt eine weitere bevorzugte Ausführungsform
·der Anschläge 26/27 für eine Kolben-Zylinderkombination,
wobei wieder ein Nocken 27 mit dem Kolben 24 verbunden ist und in einen Schlitz im Zylinder 23 gleitet.
Die Anschläge werden hierbei jedoch durch Stufen 26 im
Schlitz gebildet, so daß jeweils dann eine neue Applikation möglich ist, wenn der Kolben 24 zum Zylinder 23
verdreht wird.

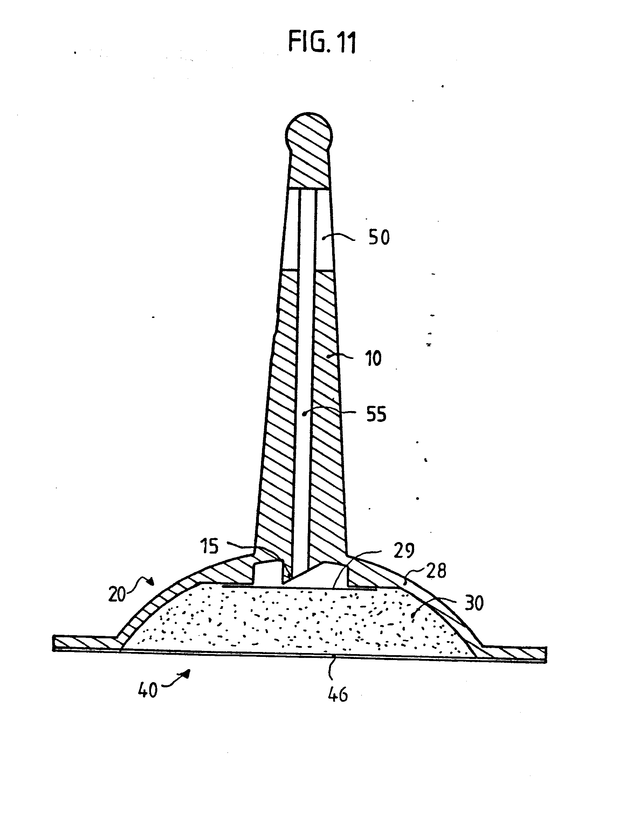

Eine weitere bevorzugte Ausführungsform der erfindungsgemäßen Vorrichtung ist in Figur 11 dargestellt. Hierbei
ist der Applikator 10 mit einer flachen Schale 28 verbunden, die an ihrem planen Hinterende mit einer Membran
46 verschlossen ist. Die Membran 46 stellt die Betätigungsmittel 40 dar. An der dem Applikator 10 zugewandten
Seite ist die flache Schale 28 mit einer dünnen, leicht
zerreißbaren Folie 29 verschlossen, die kurz über einer
Schneidfläche 15 plaziert ist, die vom Applikator 10
hervorragt. Auf diese Weise kann man die Vorrichtung
mit der Wirkstoffzubereitung 30 befüllen, ohne die Austrittsöffnungen 50 verschließen zu müssen. Wenn man bei
Anwendung die Membran 46 niederdrückt, so preßt sich
die Folie 29 gegen die Schneidfläche 15 und zerreißt,
so daß die Wirkstoffzubereitung 30 durch den Kanal 55
zu den Austrittsöffnungen 50 gelangen kann. Selbstverständlich muß man die Membran 46 wesentlich reißfester
als die Membran 29 ausbilden. Vorzugsweise wird die Schale
28 in ihrem Querschnitt leicht oval ausgebildet, so daß eine raumsparende Verpackung und Stapelung möglich ist.

Es versteht sich von selbst, daß die oben beschriebenen Ausführungsformen auch kombiniert werden können; zum Beispiel ist die in den Figuren 5, 8 und 9 gezeigte Ausbildung von Vertiefungen 13 und 14 auf der Mantelfläche des Applikators 10 auch bei den Applikatoren nach den Figuren 1, 4, 6 und 11 von Vorteil. Ebenso kann man irreversibel zu öffnende Verschlußvorrichtungen 29 (wie in Figur 11 gezeigt) bei allen Applikatoren vorsehen. Weiterhin ist es auch möglich, statt einer Zerreiß-Membran 29 Ventilvorrichtungen mit Sitz und Kolben anzubringen, wie sie aus dem Stand der Technik aus den Gebieten der Hydraulik und Pneumatik in großer Vielzahl bekannt sind (Sicherheitsventile etc). Die Anordnung der Vertiefungen 13, 14 und der Austrittsöffnungen 50 auf dem Applikator 10 ist selbstverständlich nicht nur auf die lineare, in Applikatorlängsrichtung verlaufende Form beschränkt. Es ist auch möglich, die Vertiefungen (gegebenenfalls mit Austrittsöffnungen 50) schrauben- oder schlangenlinienförmig auf dem Applikator verlaufen zu lassen. Ebenso muß der Applikator nicht unbedingt kegelförmig sein, auch zylindrische oder keulenförmige Applikatoren mit glatten oder gewellten Flächen bringen Vorteile mit sich. Bei einer weiteren bevorzugten Ausführungsform der erfindungsgemäßen Vorrichtung, insbesondere bei einer Vorrichtung nach den Figuren 1, 3, 6, 7 und 11 wird die Austrittsöffnung 50 nicht auf der Mantelfläche des Applikators, sondern an dessen Spitze ausgebildet. Derartige Vorrichtungen eignen sich dann zur rektalen Applikation von Wirkstoffen, die in den Blutkreislauf gelangen sollen, also als Ersatz von Suppositorien, falls eine Formulierung der Wirkstoffzubereitung als Suppositorien unmöglich oder unvorteilhaft ist.

71/ka

M/23 221

P a t e n t a n s p r ü c h e

1. Vorrichtung zur intra-analen Applikation von flüssigen oder pastösen Wirkstoffzubereitungen, mit einem im wesentlichen schlank-kegelförmigen Applikator mit Austrittsöffnungen,

d a d u r c h   g e k e n n z e i c h n e t , daß der Applikator (10) mit einem Behälter (20) für die zu applizierende Wirkstoffzubereitung (30) verbunden ist und Betätigungsmittel (40) aufweist, mit denen die Wirkstoffzubereitung (30) aus den Austrittsöffnungen (50) gepreßt wird.

2. Vorrichtung nach Anspruch 1, dadurch gekennzeichnet, daß der Behälter (20) als Tube (21) mit zusammengequetschtem Abschlußrand (22) ausgebildet und mit dem Applikator (10) einstückig aus Kunststoffmaterial geformt ist.

3. Vorrichtung nach Anspruch 1, dadurch gekennzeichnet, daß der Behälter (20) als Zylinder (23) ausgeführt ist, der durch einen Kolben (24) verschlossen ist, an dem die Betätigungsmittel ansetzen.

4. Vorrichtung nach Anspruch 3, dadurch gekennzeichnet, daß die Betätigungsmittel (40) fest, vorzugsweise einstückig mit dem Kolben (24) verbunden sind.

5. Vorrichtung nach Anspruch 4, dadurch gekennzeichnet, daß an Kolben (24) und Zylinder (23) korrespondierende Anschläge (26;27) vorgesehen sind, die den Weg des Kolbens (24) im Zylinder (23) auf den der wirksamen Dosis der zu applizierenden Wirkstoffzubereitung entsprechenden Hub begrenzen.

6. Vorrichtung nach einem der Ansprüche 1 bis 4, dadurch gekennzeichnet, daß der Behälter (20) ein Volumen aufweist, das einer wirksamen Dosis der zu applizierenden Wirkstoffzubereitung entspricht.

7. Vorrichtung nach Anspruch 3, dadurch gekennzeichnet, daß die Betätigungsmittel (40) als Vorrichtungen ausgebildet sind, die mit dem Applikator (10) lösbar verbunden werden können, ohne bei Betätigung mit der Wirkstoffzubereitung (30) in Kontakt zu gelangen.

8. Vorrichtung nach Anspruch 7, dadurch gekennzeichnet, daß die Betätigungsmittel (40) einen Stempel (41) mit Daumenauflage (42) umfassen, der in einem Rohr (43) mit Fingerauflage (44) geführt ist, daß das Rohr (43) Verbindungsmittel, vorzugsweise ein Gewinde (45), aufweist, über die der Applikator (10) mit korrespondierenden Verbindungsmitteln (11) angekoppelt werden kann, und daß der Zylinder (43) im Applikator (10) angebracht und durch den als Stopfen ausgebildeten Kolben (24) verschlossen ist.

9. Vorrichtung nach Anspruch 1, dadurch gekennzeichnet, daß der Behälter (20) als flache Schale (28) ausgebildet ist, die mit den Betätigungsmitteln (40) in Form einer weichen, deformierbaren Membran (46) verschlossen und mit dem Applikator (10) einstückig verbunden ist, wobei vorzugsweise Dichtmittel (29) vorgesehen sind, die den mit der Wirkstoffzubereitung (30) gefüllten Innenraum der Schale (28) von den Austrittsöffnungen (50) des Applikators (10) trennen, und daß Mittel (15) vorgesehen sind, welche bei Betätigung der Betätigungsmittel (40) die Dichtmittel (29) irreversibel öffnen.

10. Vorrichtung nach einem der vorhergehenden Ansprüche, dadurch gekennzeichnet, daß die Austrittsöffnungen (50) des Applikators (10) auf dessen im wesentlichen kegelförmiger Mantelfläche vorgesehen sind, und daß die Mantelfläche mindestens in diesen Bereichen Abflachungen (13) oder Vertiefungen zum erleichterten Austritt der Wirkstoffzubereitung aufweisen.

11. Vorrichtung nach einem der vorhergehenden Ansprüche, dadurch gekennzeichnet, daß in der Längsrichtung des Applikators (10) eine Vielzahl von Austrittsöffnungen (50a, 50b, 50c) vorgesehen sind, deren Öffnungsweiten zur Spitze des Applikators (10) hin zunehmen.

FIG.1

FIG.2

2/7

0139855

FIG. 3

FIG.4

FIG. 5

FIG.6

FIG.7

55
50a
50b
50c
23
10
30
24
11

45
40
43
44
41
42

FIG. 8

13    -55-    10
14

FIG. 9

14
10
13    -55-
50

FIG. 10

-23-
26
27
24    44

FIG. 11

0139855

**Europäisches Patentamt**

# EUROPÄISCHER RECHERCHENBERICHT

Nummer der Anmeldung

## EINSCHLÄGIGE DOKUMENTE

EP 84107862.9

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | US - A - 3 225 763 (N.S. WATERMAN) | 1,6,10, 11 | A 61 M 31/00 |
| Y | * Gesamt * | 2 | |
| | -- | | |
| Y | FR - A - 1 239 657 (M.R. SCHENKER) | 2 | |
| | * Gesamt; insbesondere Seite 2, 5. Absatz; Fig. 2 * | | |
| | -- | | |
| X | US - A - 2 848 998 (H.D. BRYAN) | 1,3,4, 6 | |
| Y | * Gesamt | 5,7 | |
| | -- | | |
| Y | US - A - 2 826 198 (W.E. VAN SICKLE) | 5 | |
| | * Fig. 6; Spalte 3, Zeilen 18-23 * | | RECHERCHIERTE SACHGEBIETE (Int. Cl.4) |
| | -- | | A 61 M 31/00 |
| Y | FR - A - 1 178 160 (LABORATOIRES THERSCIA) | 7 | |
| A | * Gesamt * | 8 | |
| | -- | | |
| X | US - A - 3 154 074 (T.S. HARRISON) | 1,6 | |
| A | * Gesamt * | 9 | |
| | -- | | |
| X | US - A - 2 664 088 (J. HOCH) | 1,6 | |
| | * Gesamt * | | |
| | -- | | |

Der vorliegende Recherchenbericht wurde für alle Patentansprüche erstellt.

| Recherchenort | Abschlußdatum der Recherche | Prüfer |
|---|---|---|
| WIEN | 28-11-1984 | LUDWIG |

**0139855**

EUROPÄISCHER RECHERCHENBERICHT

Nummer der Anmeldung

Europäisches
Patentamt

| EINSCHLÄGIGE DOKUMENTE | | | EP 84107862.9 |
|---|---|---|---|

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| P,X | FR - A - 2 522 506 (LABORATOIRES BIOTHERAX) <br> * Gesamt * <br> ---- | 1,3,4, 6 | |
| | | | **RECHERCHIERTE SACHGEBIETE (Int. Cl.4)** |

Der vorliegende Recherchenbericht wurde für alle Patentansprüche erstellt.

| Recherchenort | Abschlußdatum der Recherche | Prüfer |
|---|---|---|
| WIEN | 28-11-1984 | LUDWIG |